Europäisches Patentamt

European Patent Office

Office européen des brevets

(11) Publication number : 0 495 743 A1

# EUROPEAN PATENT APPLICATION

(21) Application number : 92610003.3

(22) Date of filing : 15.01.92

(51) Int. Cl.⁵ : A61M 35/00, A61F 13/02, A61K 9/70

(30) Priority : 17.01.91 DK 82/91

(43) Date of publication of application :
22.07.92 Bulletin 92/30

(84) Designated Contracting States :
AT BE CH DE DK ES FR GB GR IT LI LU MC NL
PT SE

(72) Inventor : Berthelsen, Viggo
Lundely 14
dk-2900 Hellerup (DK)

(74) Representative : Bagger-Soerensen, Birgitte et al
c/o Th. Ostenfeld Patentbureau A/S Bredgade
41 P.O. Box 1183
DK-1011 Copenhagen K (DK)

(71) Applicant : Berthelsen, Viggo
Lundely 14
dk-2900 Hellerup (DK)

(54) A composition comprising an active ingredient, preferably being powdery or particulate, and a carrier for said active ingredient as well as a method for the preparation of such composition.

(57) A composition (1) comprising an active ingredient (5), preferably being powdery or particulate, as well as a carrier for the active ingredient, using as carrier two sheets of material (10, 20), one of said sheets (10) on one surface being provided with a plurality of closed loops (11), and the other sheet (20) on one surface being provided with a plurality of hooks (21), said hooks being able to catch the closed loops on the other sheet when the two sheets of material are pressed together with said surfaces facing each other, whereby the two sheets may be attached to each other, said active ingredient (5) being distributed between the surfaces facing each other, and said hooks (21) and said loops (11) being engaged with each other by pressing said sheets (10, 20) together, whereby the active ingredient is kept in position between said hooks and said loops.

The composition is prepared by distributing in a desired pattern the active ingredient on said surface of one of said sheets being provided with loops or hooks and pressing together the two sheets of material with the surfaces provided with loops and hooks, respectively, facing each other.

Fig. 1

This invention relates to a composition comprising an active ingredient, preferably being powdery or particulate, as well as a carrier for the active ingredient. Furthermore, the invention relates to a method for the preparation of such composition and to the use of two sheets of material, one of said sheets on one surface being provided with a plurality of closed loops, and the other sheet on one surface being provided with a plurality of hooks, said hooks being able to catch the closed loops on the other sheet when the two sheets of material are pressed together with said surfaces facing each other, whereby the two sheets may be attached to each other, as a carrier for an active ingredient, said active ingredient preferably being powdery or particulate.

This invention is of particular use in connection with active ingredients, the activity of which it is desired to transfer to an object, e.g. the human body, over a certain period of time, e.g. some hours or more. The transfer of activity may e.g. take place by radiation, diffusion or in other ways.

Among the active ingredients, the activity of which it may be desirable to transfer in this manner, are parts of plants having grown on mineral-enriched or mineral-rich soil and/or parts of plants and minerals being powdery or particulate, the mineral spectrum thereof being very broad.

Previously, this active ingredient has been incorporated into an ointment base and applicated to the human body in this form. The duration of the effect has been regulated by varying the thickness of the ointment layer, and in order to prevent the ointment layer from smudging, it has been covered by a piece of plastic foil. Furthermore, as the transfer of activity from the active ingredient does not require direct contact of said active ingredient with the body, attempts have been made to place the ointment containing the active ingredient as a layer between two pieces of plastic foil being welded together along their edges, and place said laminate of plastic foil and ointment with active ingredient in contact with the body. However, this structure suffers from the disadvantage that the body heat will cause the ointment to run and thus make it impossible to maintain the desired uniform distribution of the active ingredient.

According to the present invention this problem is solved by using as carrier for an active ingredient, preferably being powdery or particulate, two sheets of material, one of said sheets on one surface being provided with a plurality of closed loops, and the other sheet on one surface being provided with a plurality of hooks, said hooks being able to catch the closed loops on the other sheet, when the two sheets of material are pressed together with said surfaces facing each other, whereby the two sheets may be attached to each other.

Accordingly, the present invention provides a composition of the kind stated in the introductory part of claim 1, said composition being characterized by the subject matter stated in the characterizing part of claim 1.

Such respective sheets of material are commercially available and are typically in the shape of woven ribbons. One material of this type is available under the trademark "Velcro".

In one embodiment of the composition according to the invention the two sheets of material are substantially of the same extension.

In a preferred embodiment the composition according to the invention takes the form of a comparatively rigid structure intended for being brought into contact with an object in order to transfer its activity thereto. The rigidity may be provided by one of said sheets of material or both and/or by the formation of a sandwich structure with one or more sheets of comparatively rigid material such as paperboard, cardboard or plastic material. The structure may be surrounded by a cover or wrap, e.g. a plastic bag.

In another preferred embodiment of the composition according to the invention the two sheets of material take the form of a flexible, elongated structure adapted to embrace a part of the body, e.g. in a belt- or bracelet-like fashion. A structure of this kind may be fastened e.g. by means of a buckle or clasp or the like.

In one embodiment of the composition according to the invention the two sheets of material are mutually staggered, the free ends of the sheets of material thereby being usable for fastening e.g. of an elongated structure as mentioned above.

Furthermore, the present invention relates to a method of the kind stated in the introductory part of claim 8, said method being characterized by the subject matter stated in the characterizing part of claim 8.

When carrying out the method according to the invention the active ingredient will typically be distributed on the surface provided with hooks, said surface usually being the most open, but there is no obstacle to distributing the active ingredient on the surface provided with loops instead.

In one embodiment of the method according to the invention the two sheets of material are in advance cut into suitable pieces for the formation of a unit. In an alternative embodiment the two sheets of material are cut into suitable pieces for the formation of a unit after application of the active ingredient and pressing together of the two sheets.

In order to avoid loss of active ingredient said active ingredient is preferably distributed in a pattern so as to be present only in a certain distance from the edge of the unit formed.

Preferably the active ingredient comprises parts of plants having grown on mineral-enriched or mineral-rich soil and/or parts of plants and minerals in powdery or particulate form. As an example, dolomite having a high content of trace minerals, may be used as mineral source. Also coal and fly ash originating from coal may be used.

Preferably the active ingredient is powdery or particulate, but of course there is no obstacle to using said carrier as a carrier for an active ingredient in liquid form as well or a mixture of liquid and solid form, if desired.

In the following the invention will be described in more detail, with reference to the drawings, wherein

fig. 1 is schematic cross-sectional view showing the structure of a composition according to the invention,

fig. 2 is a schematic view of an embodiment of a composition according to the invention adapted to embrace a part of the body, and

fig. 3 is a schematic view of an embodiment of a composition according to the invention in the shape of a comparatively rigid structure.

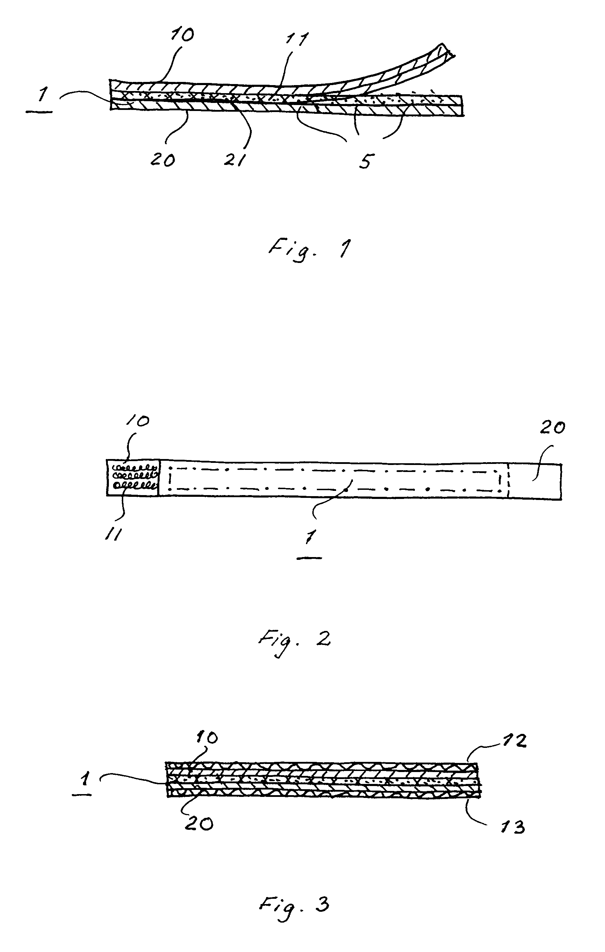

Fig. 1 is a schematic cross-sectional view of a composition 1 according to the invention comprising a carrier in the form of two sheets of material 10, 20, one of said sheets 10 on the surface facing the sheet 20 being provided with a plurality of closed loops 11 (depicted schematically as a cohesive layer), and the other sheet 20 on the surface facing the sheet 10 being provided with a plurality of hooks 21 (depicted schematically as a cohesive layer). On the left-hand side of the figure the two sheets have been pressed together in such manner that the loops 11 and the hooks 21 have engaged each other, and it is seen how the active ingredient 5 in the engagement zone is distributed and fixed between the hooks and the loops, whereas the active ingredient 5 on the right-hand side of the figure still lies loosely between and upon the hooks 21. In the depicted embodiment the two sheets of material are substantially coextending.

Fig. 2 shows a composition 1 according to the invention, wherein the carrier is formed by two sheets of material 10, 20 in the shape of woven ribbons. The composition is shaped as an elongated structure, and the two sheets of material are mutually staggered in the longitudinal direction, sheet 10 on the left-hand side of the figure having a freely protuding end, where the closed loops 11 may be seen. Correspondingly, the sheet of material 20 has a freely protuding end on the right-hand side of the figure, where the hooks protude on the side facing away from the observer. The active ingredient is present within the area marked by the dash-dot line. The elongated structure may be wrapped around a part of the body, e.g. a wrist, and fastened by pressing the protuding ends of the sheets of material 10, 20 against each other with the loops 11 and the hooks 21 facing each other.

Fig. 3 is a schematic view of a composition similar to that in fig. 1, but with the two sheets of material 10, 20 being embedded between two pieces of corrugated paper 12, 13 to form a comparatively rigid structure. The resulting sandwich structure may be surrounded by a cover of plastic foil or the like, e.g. in the form of a plastic bag (not shown).

In the foregoing the invention has been described by means of specific embodiments, however, it is obvious that various modifications and alterations may be carried out without departing from the spirit and scope of the invention.

**Claims**

1. A composition (1) comprising an active ingredient (5), preferably being powdery or particulate, as well as a carrier for the active ingredient, CHARACTERIZED in using as carrier two sheets of material (10, 20), one of said sheets (10) on one surface being provided with a plurality of closed loops (11), and the other sheet (20) on one surface being provided with a plurality of hooks (21), said hooks being able to catch the closed loops on the other sheet when the two sheets of material are pressed together with said surfaces facing each other, whereby the two sheets may be attached to each other, said active ingredient (5) being distributed between the surfaces facing each other, and said hooks (21) and said loops (11) being engaged with each other by pressing said sheets (10, 20) together, whereby the active ingredient is kept in position between said hooks and said loops.

2. A composition as claimed in claim 1, CHARACTERIZED in that the two sheets of material (10, 20) are substantially coextending.

3. A composition as claimed in claims 1 or 2, CHARACTERIZED in that the two sheets of material (10, 20) are in the shape of woven ribbons.

4. A composition as claimed in one or more of the preceeding claims, CHARACTERIZED in being formed as a comparatively rigid structure, optionally by embedding in a sandwich structure with one or more sheets (12, 13) of a comparatively rigid material.

5. A composition as claimed in one or more of the preceeding claims, CHARACTERIZED in that the two sheets of material (10, 20) are formed as a flexible, elongated structure adapted to embrace a part of the body, e.g. in a belt- or bracelet-like fashion.

6. A composition as claimed in one or more of the preceeding claims, CHARACTERIZED in that the two sheets of material (10, 20) are mutually staggered.

7. A composition as claimed in one or more of the preceeding claims, CHARACTERIZED in comprising as an active ingredient (5) parts of plants having grown on mineral-enriched or mineral-rich soil, and/or parts of plants and minerals being powdery or particulate.

8. A method for preparing a composition (1) comprising an active ingredient (5), preferably being powdery or particulate, as well as a carrier for the active ingredient, CHARACTERIZED in providing two sheets of material (10, 20), one of said sheets (10) on one surface being provided with a plurality of closed loops (11), and the other sheet (20) on one surface being provided with a plurality of hooks (21), said hooks being able to catch the closed loops on the other sheet when the two sheets of material are pressed together with said surfaces facing each other, whereby the two sheets may be attached to each other, distributing said active ingredient (5) on said surface on one of said sheets (10, 20) in a desired pattern and pressing the second sheet against the first one with said surfaces facing each other, thus keeping the active ingredient in position between said hooks and said loops.

9. A method as claimed in claim 8, CHARACTERIZED in that the two sheets of material (10, 20) are pre-cut into suitable pieces for the formation of a unit.

10. A method as claimed in claim 8, CHARACTERIZED in that the two sheets of material (10, 20) are cut into suitable pieces for the formation of a unit after introduction of the active ingredient (5), and pressing together the two sheets.

11. A method as claimed in claims 9 or 10, CHARACTERIZED in distributing the active ingredient (5) in a pattern so as to be present only in a certain distance from the edge of the unit formed.

12. The use of two sheets of material (10, 20), one of said sheet (10) on one surface being provided with a plurality of closed loops (11), and the other sheet (20) on one surface being provided with a plurality of hooks (21), said hooks being able to catch the closed loops on the other sheet when the two sheets of material are pressed together with said surfaces facing each other, whereby the two sheets may be attached to each other, as carrier for an active ingredient (5), preferably being powdery or particulate.

*10*  *11*

*1*

*20*  *21*  *5*

Fig. 1

*10*  *20*

*11*  *1*

Fig. 2

*10*  *12*

*1*

*20*  *13*

Fig. 3

European Patent Office

**EUROPEAN SEARCH REPORT**

Application Number

EP 92 61 0003

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 910 020 (SAMOUR)<br>--- | 1-12 | A61M35/00<br>A61F13/02<br>A61K9/70 |
| A | GB-A-2 150 028 (NOLAN)<br>--- | 1-12 | |
| A | FR-A-2 196 589 (OPEN S.A.)<br>--- | 1-12 | |
| A | US-A-4 689 230 (AYOUB) | 1-12 | |
| | ----- | | |
| | | | **TECHNICAL FIELDS SEARCHED (Int. Cl.5)**<br><br>A61K<br>A61M<br>A61F |

The present search report has been drawn up for all claims

| Place of search | Date of completion of the search | Examiner |
|---|---|---|
| THE HAGUE | 27 APRIL 1992 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)